# EUROPÄISCHE PATENTANMELDUNG

(11) **EP 4 001 254 A1**
(43) Veröffentlichungstag der Anmeldung: **25.05.2022**
(21) Anmeldenummer: 20209158.3
(22) Anmeldetag: 23.11.2020
(51) Int. Cl.: C07C 67/38, C07C 69/14

(54) **RU-KATALYSIERTE DOMINO HYDROFORMYLIERUNG/HYDRIERUNG/VERESTERUNG UNTER EINSATZ VON IMIDAZOL-LIGANDEN**

(71) Anmelder: Evonik Operations GmbH, 45128 Essen (DE)
(72) Erfinder: FRANKE, Robert, 45772 Marl (DE); KUCMIERCZYK, Peter, 44628 Herne (DE); BELLER, Matthias, 18211 Ostseebad Nienhagen (DE); DÜHREN, Ricarda, 18069 Rostock (DE); JACKSTELL, Ralf, 18106 Rostock (DE)
(74) Vertreter: Evonik Patent Association

(57) **Zusammenfassung**

Ru-katalysierte Domino Hydroformylierung/Hydrierung/Veresterung unter Einsatz von Imidazol-Liganden.

## Beschreibung

Die vorliegende Erfindung betrifft eine Ru-katalysierte Domino Hydroformylierung/Hydrierung/Veresterung unter Einsatz von Imidazol-Liganden.

In EP 0 329 252 B1 wird ein Verfahren zur Herstellung von Carbonsäuren oder Estern beschreiben. Das Verfahren wird hierbei durch den Einsatz einer Rutheniumverbindung in Kombination mit einer sauren Verbindung katalysiert. Die saure Verbindung umfasst hierbei eines der folgenden Elemente: Phosphor, Antimon, Arsen, Molybdän, Wolfram.

In I. Fleischer, K. M. Dyballa, R. Jennerjahn, R. Jackstell, R. Franke, A. Spannenberg, M. Beller, "From Olefins to Alcohols: Efficient and Regioselective Ruthenium-Catalyzed Domino Hydroformylation/Reduction Sequence", Angew. Chem. Int. Ed. 2013, 52, 2949 - 2953 wir ein Verfahren zur Hydroformylierung und anschließender Reduktion beschrieben. Das eingesetzte Olefin wird hierbei zum Aldehyd umgesetzt und abschließend zum Alkohol reduziert.

Die technische Aufgabe, welche der vorliegenden Erfindung zugrunde lag, ist die Bereitstellung eines Verfahrens, welches ausgehend von einem Olefin direkt zu einem Ester führt, ohne dass Zwischenstufen isoliert werden. Wobei das ursprüngliche Olefin den Alkoholteil des Esters ausbilden soll, d.h. über den Sauerstoff an das Kohlenstoffatom der C=O-Gruppe gebunden ist. Des Weiteren soll der Alkoholteil des Esters über einen Kohlenstoff mehr verfügen, als das eingesetzte Olefin.

Diese Aufgabe wird gelöst durch ein Verfahren gemäß Anspruch 1.

Verfahren umfassend die Verfahrensschritte:
a) Vorlegen einer ethylenisch ungesättigten Verbindung;
b) Zugabe eines Liganden gemäß der Formel (I): 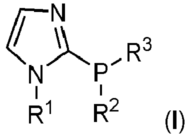 wobei
   R¹ ausgewählt ist aus: -(C₁-C₁₂)-Alkyl, -(C₆-C₁₂)-Cycloalkyl, -(C₆-C₂₀)-Aryl, -(C₅-C₂₀)-Heteroaryl,
   wobei der -(C₆-C₁₂)-Cycloalkyl-Rest, der -(C₆-C₂₀)-Aryl-Rest und der -(C₅-C₂₀)-Heteroaryl-Rest Substituenten aufweisen können, welche ausgewählt sind aus: -(C₁-C₁₂)-Alkyl, -O-(C₁-C₁₂)-Alkyl;
   R² und R³ ausgewählt sind aus: -(C₁-C₁₂)-Alkyl, -(C₆-C₁₂)-Cycloalkyl, -(C₆-C₂₀)-Aryl; wobei der -(C₆-C₁₂)-Cycloalkyl-Rest, und der -(C₆-C₂₀)-Aryl-Rest Substituenten aufweisen können, welche ausgewählt sind aus: -(C₁-C₁₂)-Alkyl, -O-(C₁-C₁₂)-Alkyl; und einer Verbindung, welche Ru umfasst;
c) Zugabe einer Säure (**II**), welche die Formel (**IIa**) oder (**IIb**) aufweist: 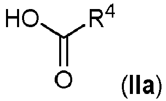 wobei R⁴ für -(C₁-C₁₈)-Alkyl steht; 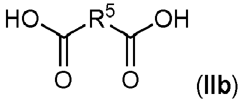 wobei R⁵ für -(C₁-C₁₈)-Alkyl steht;
d1) Zuführen von CO;
e) Erwärmen des Reaktionsgemisches aus a) bis d), wobei die ethylenisch ungesättigte Verbindung direkt zu einem Ester beziehungsweise Diester der Säure (**II**) umgesetzt wird, ohne dass Zwischenstufen isoliert werden.

Hierbei umfasst d) den Verfahrensschritt d1) und gegebenenfalls die weiteren Verfahrensschritte d2), d3) und d4).

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Der Begriff (C₁-C₁₂)-Alkyl umfasst geradkettige und verzweigte Alkylgruppen mit 1 bis 12 Kohlenstoffatomen. Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Der Begriff (C₆-C₁₂)-Cycloalkyl umfasst mono-, bi- oder tricyclische Kohlenwasserstoffgruppen.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₅-C₂₀)-Heteroaryl umfasst mono- oder polycyclische aromatische Kohlenwasserstoffreste mit 5 bis 20 Kohlenstoffatomen, wobei eins oder mehrere der Kohlenstoffatome durch Heteroatome ersetzt sind. Bevorzugte Heteroatome sind N, O und S. Die (C₅-C₂₀)-Heteroarylgruppen weisen 5 bis 20, bevorzugt 5 bis 6 Ringatome auf. Somit ist beispielsweise Pyridyl im Rahmen dieser Erfindung ein C₆-Heteroarylrest, Furyl ist ein C₅-Heteroarylrest.

Geeignete (C₅-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein. Vorzugsweise weist die ethylenisch ungesättigten Verbindungen eine Kohlenstoff-Kohlenstoff-Doppelbindungen auf.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform ist die ethylenisch ungesättigte Verbindung ausgewählt aus: Ethen, Propen, 1-Buten, cis und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.
In einer Ausführungsform ist R¹ ausgewählt aus: -(C₁-C₁₂)-Alkyl, -(C₆-C₂₀)-Aryl, -(C₅-C₂₀)-Heteroaryl.

In einer Ausführungsform stehen R² und R³ für -(C₃-C₁₂)-Cycloalkyl.

In einer Ausführungsform stehen R² und R³ für Cy.

In einer Ausführungsform ist die Verbindung, welche Ru umfasst, ausgewählt aus: Ru₃(CO)₁₂, RuCl₃*H₂O, Ru(Cl)₂(DMSO)₄, Ru(acac)₃.

In einer Ausführungsform ist die Verbindung, welche Ru umfasst, Ru₃(CO)₁₂.

In einer Ausführungsform steht R⁴ für -(C₁-C₁₂)-Alkyl.

In einer Ausführungsform steht R⁴ für -(C₁-C₈)-Alkyl.

In einer Ausführungsform steht R⁵ für -(C₁-C₁₂)-Alkyl.

In einer Ausführungsform steht R⁵ für -(C₁-C₈)-Alkyl.

In einer Ausführungsform umfasst das Verfahren den zusätzlichen Verfahrensschritt d2): d2) Zuführen von H₂.

In einer Ausführungsform liegt der H₂-Druck im Bereich von 1 MPa (10 bar) bis 6 MPa (60 bar).

In einer Ausführungsform umfasst das Verfahren den zusätzlichen Verfahrensschritt d3): d3) Zugabe von H₂O.

In einer Ausführungsform wird H₂O in einer Menge zugegeben, so dass das molare Verhältnis von H₂O zu der ethylenisch ungesättigten Verbindung im Bereich von 1:1 bis 10:1 liegt.

In einer Ausführungsform umfasst das Verfahren den zusätzlichen Verfahrensschritt d4): d4) Zugabe von para-Toluolsulfonsäure.

In einer Ausführungsform wird die Säure (**II**) in Verfahrensschritt c) in einer Menge zugegeben, so dass das molare Verhältnis von Säure zu der ethylenisch ungesättigten Verbindung im Bereich von 2:1 bis 10:1 liegt.

In einer Ausführungsform weist die Säure (**II**) die Formel (**IIa**) auf.

In einer Ausführungsform weist die Säure (**II**) die Formel (**IIb**) auf.

In einer Ausführungsform ist der Ligand in Verfahrensschritt b) ausgewählt aus:

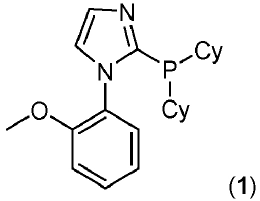

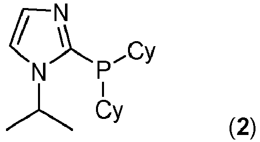

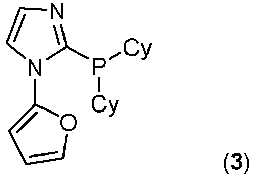

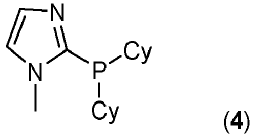

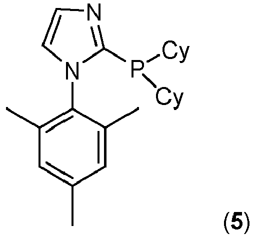

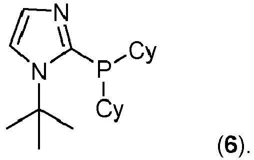

In einer Ausführungsform wird die Reaktionsmischung im Verfahrensschritt e) auf eine Temperatur zwischen 50 °C und 180 °C, bevorzugt zwischen 80 °C und 160 °C, besonders bevorzugt zwischen 100 °C und 150 °C erwärmt, um die ethylenisch ungesättigte Verbindung zum Ester umzusetzen.

Im Folgenden wird die Erfindung anhand von Ausführungsbeispielen näher beschrieben.

### Allgemeine Arbeitsvorschriften

Alle Arbeiten mit luft- und feuchtigkeitsempfindlichen Stoffen wurden in einer Argonatmosphäre und mit ausgeheizten Glasgeräten unter Verwendung der Schlenk-Technik durchgeführt. Die Chemikalien wurden von kommerziellen Herstellern bezogen und so verwendet wie sie geliefert wurde, wenn die Reinheit mindestens 98% betrug. Sauerstofffreie und trockene Lösungsmittel wurden mittels Destillation unter Argon vorbereitet. Synthesegas (CO:99,997%, H₂/CO:1:1+/-1%) wurde von der Firma Linde bezogen.

Die Produkte wurden mittels ¹H-NMR- und ¹³C-NMR-Spektroskopie analysiert. Die NMR-Spektren wurden auf Geräten des Typs Bruker AV 400 (400 MHz), Bruker AV 300 (300 MHz), oder Fourier 300 (MHz) aufgenommen. Chemische Verschiebungen δ (ppm) werden relativ zum verwendeten Lösungsmittel angegeben: Referenzen für CDCl₃ waren 7.26 ppm (¹H-NMR) und 77.16 (¹³C-NMR). ¹³C-NMR-Spekren wurden mit einer breitbandentkoppelnden Methode aufgenommen.

GC-Analysen wurden auf einem 7890A GC-System der Firma Agilent Technologies mit einer 30 m HP-5 Säule durchgeführt. Als Trägergas wurde Argon verwendet. Die Produkte wurden mittels GC oder GC-MS analysiert oder mittels Säulenchromatographie (Silika, EtOAc/Heptan) isoliert. Die GC-Ausbeuten wurden über interne Kalibrierung berechnet. Als interner Standard wurde Hexadecan verwendet.

### Durchführung der katalytischen Experimente

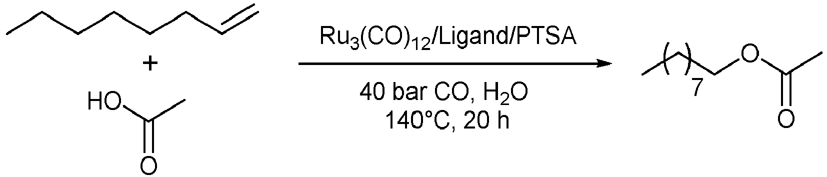

Die katalytischen Experimente wurden in 4 mL Glasvials mit Schraubverschlusskappen und einem PTFE-Septum durchgeführt. Die Vials wurden mit ofengetrockneten Magnetrührern bestückt und über eine Nadel die Verbindung zur Gasatmosphäre hergestellt. Der Reaktionsansatz betrug 2 mL. Die Vials wurden in einem 300 mL Parr4560 Autoklav platziert und über einem Magnetrührwerk gerührt. Im ersten Schritt werden Ru₃(CO)₁₂ (5mol%), Ligand (5.5mol%) und PTSA*H₂O (20.6 mol%) in das Vial eingewogen. Das Vial wird mit einer Schraubverschlusskappe, die mit einem Septum versehen ist, verschlossen und über eine Kanüle mit der Argonatmosphäre verbunden. Das Vial wurde dreimal sekuriert und mit Argon gespült. Essigsäure (1.17 mL), H₂O (0.35 mL) und 1-Octen (3 mmol) wurde mittels Hamiltonspritze injiziert. Unter Argonatmosphäre wurde das Vial in den Autolaven überführt. Der Autoklav wird fest verschlossen und zunächst bei Raumtemperatur dreimal mit 10 bar CO gespült. Danach werden 40 bar CO aufgepresst, der Autoklav in einem Aluminiumblock auf einem Magnetrührwerk platziert und für 20 h auf 140 °C geheizt. Nach 20 h wurde der Autoklav auf Raumtemperatur heruntergekühlt und der Druck vorsichtig abgelassen. Als interner Standard wurden 100 µL Hexadecan in die Reaktionslösung gegeben. Die Ausbeute wurden mittels GC-Analyse bestimmt.

Die Reaktion wurde unter analogen Bedingungen für die Liganden (1) bis (6), sowie für den Vergleichsliganden (7) durchgeführt. Da es sich bei dem Vergleichsliganden (7) um einen bidentaten Liganden handelt wurde anstelle der 1,1 mol% nur 0,55 mol% eingesetzt.

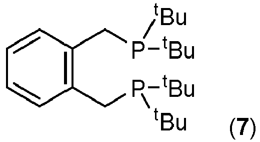

### Reaktionsbedingungen

1-Octene: 3 mmol
Ru₃(CO)₁₂: 5 mol% Ru
Ligand (**1**) bis (**6**): 5,5 mol% bezogen auf das Olefin
Ligand (**7**): 2,75 mol% bezogen auf das Olefin
PTSA*H₂O unter Verwendung von (**1**) - (**6**): 20,6 mol%
PTSA*H₂O unter Verwendung von (**7**): 10,3 mol%
H₂O : 1-Octene = 6,5 : 1 (Molverhältnis)
HOAc : 1- Octene = 6,75 : 1 (Molverhältnis)
CO-Druck: 40 bar
Temperatur: 140 °C
Reaktionszeit: 20 h

### Versuchsergebnisse

| Ligand | Ausbeute Ester [%] |
|---|---|
| (**1**)* | 28 |
| (**2**)* | 28 |
| (**3**)* | 24 |
| (**4**)* | 21 |
| (**5**)* | 25 |
| (**6**)* | 18 |
| (**7**) | 10 |

| | |
|---|---|
| * erfindungsgemäßes Verfahren | |

## Patentansprüche

1. Verfahren umfassend die Verfahrensschritte:
a) Vorlegen einer ethylenisch ungesättigten Verbindung;
b) Zugabe eines Liganden gemäß der Formel (**I**): 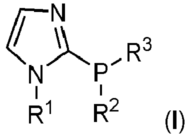 wobei
R¹ ausgewählt ist aus: -(C₁-C₁₂)-Alkyl, -(C₆-C₁₂)-Cycloalkyl, -(C₆-C₂₀)-Aryl, -(C₅-C₂₀)-Heteroaryl,
wobei der -(C₆-C₁₂)-Cycloalkyl-Rest, der -(C₆-C₂₀)-Aryl-Rest und der -(C₅-C₂₀)-Heteroaryl-Rest Substituenten aufweisen können, welche ausgewählt sind aus: -(C₁-C₁₂)-Alkyl, -O-(C₁-C₁₂)-Alkyl;
R² und R³ ausgewählt sind aus: -(C₁-C₁₂)-Alkyl, -(C₆-C₁₂)-Cycloalkyl, -(C₆-C₂₀)-Aryl; wobei der -(C₆-C₁₂)-Cycloalkyl-Rest, und der -(C₆-C₂₀)-Aryl-Rest Substituenten aufweisen können, welche ausgewählt sind aus: -(C₁-C₁₂)-Alkyl, -O-(C₁-C₁₂)-Alkyl; und einer Verbindung, welche Ru umfasst;
c) Zugabe einer Säure (**II**), welche die Formel (**IIa**) oder (**IIb**) aufweist: 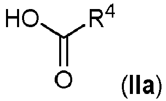 wobei R⁴ für -(C₁-C₁₈)-Alkyl steht; 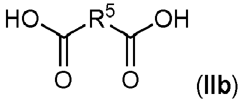 wobei R⁵ für -(C₁-C₁₈)-Alkyl steht;
d1) Zuführen von CO;
e) Erwärmen des Reaktionsgemisches aus a) bis d), wobei die ethylenisch ungesättigte Verbindung direkt zu einem Ester beziehungsweise Diester der Säure (**II**) umgesetzt wird, ohne dass Zwischenstufen isoliert werden.

2. Verfahren nach Anspruch 1,
wobei die ethylenisch ungesättigte Verbindung ausgewählt ist aus: Ethen, Propen, 1-Buten, cis und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

3. Verfahren nach einem der Ansprüche 1 oder 2,
wobei R¹ ausgewählt ist aus: -(C₁-C₁₂)-Alkyl, -(C₆-C₂₀)-Aryl, -(C₅-C₂₀)-Heteroaryl.

4. Verfahren nach einem der Ansprüche 1 bis 3,
wobei R² und R³ für -(C₃-C₁₂)-Cycloalkyl stehen.

5. Verfahren nach einem der Ansprüche 1 bis 4,
wobei die Verbindung, welche Ru umfasst, ausgewählt ist aus: Ru₃(CO)₁₂, RuCl₃*H₂O, Ru(Cl)₂(DMSO)₄, Ru(acac)₃.

6. Verfahren nach einem der Ansprüche 1 bis 5,
wobei R⁴ für -(C₁-C₁₂)-Alkyl steht.

7. Verfahren nach einem der Ansprüche 1 bis 6,
wobei R⁵ für -(C₁-C₁₂)-Alkyl steht.

8. Verfahren nach einem der Ansprüche 1 bis 7,
umfassend den zusätzlichen Verfahrensschritt d2):
d2) Zuführen von H₂.

9. Verfahren nach Anspruch 8,
wobei der H₂-Druck im Bereich von 1 MPa (10 bar) bis 6 MPa (60 bar) liegt.

10. Verfahren nach einem der Ansprüche 1 bis 9,
umfassend den zusätzlichen Verfahrensschritt d3):
d3) Zugabe von H₂O.

11. Verfahren nach Anspruch 10,
wobei H₂O in einer Menge zugegeben wird, so dass das molare Verhältnis von H₂O zu der ethylenisch ungesättigten Verbindung im Bereich von 1:1 bis 10:1 liegt.

12. Verfahren nach einem der Ansprüche 1 bis 11,
umfassend den zusätzlichen Verfahrensschritt d4):
d4) Zugabe von para-Toluolsulfonsäure.

13. Verfahren nach einem der Ansprüche 1 bis 12,
wobei die Säure (**II**) in Verfahrensschritt c) in einer Menge zugegeben wird, so dass das molare Verhältnis von Säure zu der ethylenisch ungesättigten Verbindung im Bereich von 2:1 bis 10:1 liegt.

14. Verfahren nach einem der Ansprüche 1 bis 13,
wobei die Säure (**II**) die Formel (**IIa**) aufweist.

15. Verfahren nach einem der Ansprüche 1 bis 14,
wobei der Ligand in Verfahrensschritt b) ausgewählt ist aus:

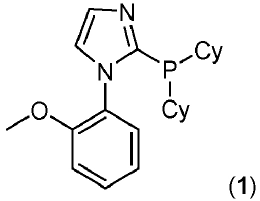

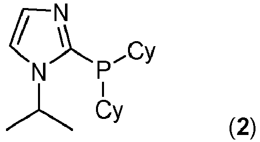

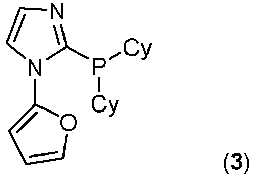

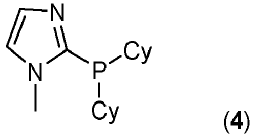

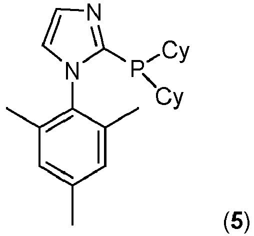

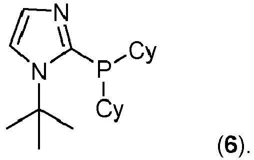

## Geänderte Patentansprüche

### Geänderte Patentansprüche gemäss Regel 137(2) EPÜ.

1. Verfahren umfassend die Verfahrensschritte:
a) Vorlegen einer ethylenisch ungesättigten Verbindung ausgewählt aus: Ethen, Propen, 1-Buten, cis und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon;
b) Zugabe eines Liganden gemäß der Formel (I): 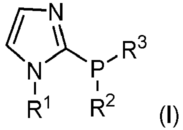 wobei
R¹ ausgewählt ist aus: -(C₁-C₁₂)-Alkyl, -(C₆-C₁₂)-Cycloalkyl, -(C₆-C₂₀)-Aryl, -(C₅-C₂₀)-Heteroaryl,
wobei der -(C₆-C₁₂)-Cycloalkyl-Rest, der -(C₆-C₂₀)-Aryl-Rest und der -(C₅-C₂₀)-Heteroaryl-Rest Substituenten aufweisen können, welche ausgewählt sind aus: -(C₁-C₁₂)-Alkyl, -O-(C₁-C₁₂)-Alkyl;
R² und R³ ausgewählt sind aus: -(C₁-C₁₂)-Alkyl, -(C₆-C₁₂)-Cycloalkyl, -(C₆-C₂₀)-Aryl; wobei der -(C₆-C₁₂)-Cycloalkyl-Rest, und der -(C₆-C₂₀)-Aryl-Rest Substituenten aufweisen können, welche ausgewählt sind aus: -(C₁-C₁₂)-Alkyl, -O-(C₁-C₁₂)-Alkyl; und einer Verbindung, welche Ru umfasst ausgewählt aus: Ru₃(CO)₁₂, RuCl₃*H₂O, Ru(Cl)₂(DmSO)₄, Ru(acac)₃;
c) Zugabe einer Säure (**II**), welche die Formel (**IIa**) oder (**IIb**) aufweist: 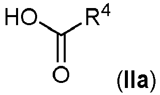 wobei R⁴ für -(C₁-C₁₈)-Alkyl steht; 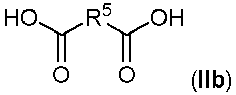 wobei R⁵ für -(C₁-C₁₈)-Alkyl steht;
d1) Zuführen von CO;
e) Erwärmen des Reaktionsgemisches aus a) bis d), wobei die ethylenisch ungesättigte Verbindung direkt zu einem Ester beziehungsweise Diester der Säure (**II**) umgesetzt wird, ohne dass Zwischenstufen isoliert werden.

2. Verfahren nach Anspruch 1,
wobei R¹ ausgewählt ist aus: -(C₁-C₁₂)-Alkyl, -(C₆-C₂₀)-Aryl, -(C₅-C₂₀)-Heteroaryl.

3. Verfahren nach einem der Ansprüche 1 oder 2,
wobei R² und R³ für -(C₃-C₁₂)-Cycloalkyl stehen.

4. Verfahren nach einem der Ansprüche 1 bis 3,
wobei R⁴ für -(C₁-C₁₂)-Alkyl steht.

5. Verfahren nach einem der Ansprüche 1 bis 4,
wobei R⁵ für -(C₁-C₁₂)-Alkyl steht.

6. Verfahren nach einem der Ansprüche 1 bis 5,
umfassend den zusätzlichen Verfahrensschritt d2): d2) Zuführen von H₂.

7. Verfahren nach Anspruch 6,
wobei der H₂-Druck im Bereich von 1 MPa (10 bar) bis 6 MPa (60 bar) liegt.

8. Verfahren nach einem der Ansprüche 1 bis 7,
umfassend den zusätzlichen Verfahrensschritt d3): d3) Zugabe von H₂O.

9. Verfahren nach Anspruch 8,
wobei H₂O in einer Menge zugegeben wird, so dass das molare Verhältnis von H₂O zu der ethylenisch ungesättigten Verbindung im Bereich von 1:1 bis 10:1 liegt.

10. Verfahren nach einem der Ansprüche 1 bis 9,
umfassend den zusätzlichen Verfahrensschritt d4): d4) Zugabe von para-Toluolsulfonsäure.

11. Verfahren nach einem der Ansprüche 1 bis 10,
wobei die Säure (**II**) in Verfahrensschritt c) in einer Menge zugegeben wird, so dass das molare Verhältnis von Säure zu der ethylenisch ungesättigten Verbindung im Bereich von 2:1 bis 10:1 liegt.

12. Verfahren nach einem der Ansprüche 1 bis 11,
wobei die Säure (**II**) die Formel (**IIa**) aufweist.

13. Verfahren nach einem der Ansprüche 1 bis 12,
wobei der Ligand in Verfahrensschritt b) ausgewählt ist aus:
